(19) Europäisches Patentamt
European Patent Office
Office européen des brevets

(11) EP 4 410 423 A1

(12) **EUROPEAN PATENT APPLICATION**

(43) Date of publication:
**07.08.2024 Bulletin 2024/32**

(21) Application number: **24154926.0**

(22) Date of filing: **31.01.2024**

(51) International Patent Classification (IPC):
***B01J 23/755*** *(2006.01)* ***B01J 35/73*** *(2024.01)*
***B01J 35/45*** *(2024.01)*

(52) Cooperative Patent Classification (CPC):
(C-Sets available)
**B01J 23/002; B01J 23/755; B01J 35/45;**
**B01J 35/615; B01J 35/73; B01J 35/77;**
**B01J 37/18; C07C 231/12;** B01J 2235/15;
B01J 2235/30 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.02.2023 US 202363442532 P**

(71) Applicant: **Academia Sinica**
**Taipei City 11529 (TW)**

(72) Inventors:
- **CHUNG, PO-WEN**
**803005 Kaohsiung City (TW)**
- **DU, YUAN-PENG**
**115201 Taipei City (TW)**

(74) Representative: **Michalski Hüttermann & Partner Patentanwälte mbB**
**Kaistraße 16A**
**40221 Düsseldorf (DE)**

(54) **BIFUNCTIONAL CATALYSTS DERIVED FROM LAYERED DOUBLE HYDROXIDES AND CONVERSION OF BETA-HYDROXY CARBONYL SPECIES AND PREPARATION OF AMINO ALCOHOL PRECURSOR USING THE SAME**

(57) Disclosed are bifunctional composite catalysts which have a basic mixed oxide support derived from layer double hydroxides and methods for conversion of β-hydroxy carbonyl species and preparation of amino alcohol precursor using the same. By the bifunctional catalyst, the abundant basic sites on hydrotalcite oxide (HTO) allow retro-aldol reaction to outpace direct hydrogenation, thus achieving an exceptional selectivity towards a desired product produced through retro-aldol reaction and then hydrogenation by, for example, nickel nanoparticles on the support. Accordingly, this method exhibits particular utility in the renewable production of N-acetylethanolamine from biomass-derived N-acetyl glucosamine (GlcNAc) without using homogeneous base as a co-catalyst.

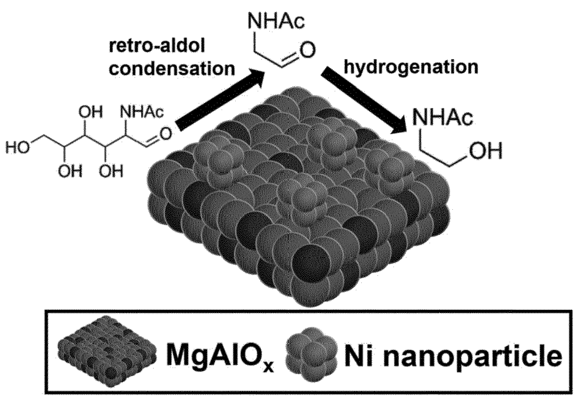


**FIG. 1**

EP 4 410 423 A1

(52) Cooperative Patent Classification (CPC): (Cont.)

C-Sets
**C07C 231/12, C07C 233/18;**
**C07C 231/12, C07C 233/31**

## Description

FIELD OF THE INVENTION

The present invention relates to bifunctional catalysts derived from layer double hydroxides and conversion of β-hydroxy carbonyl species and preparation of amino alcohol precursor using the same.

DESCRIPTION OF RELATED ART

Biomass is emerging as a promising renewable alternative to petroleum-based carbon resources due to the environmental impact caused by the production of petrochemicals from oil refineries. To date, research focuses have been mainly placed on the conversion of lignocellulosic biomass. In contrast, the valorization of marine biomass, that is the second most abundant biomass, has not been extensively studied. Marine biomass like crustacean shell is primarily composed of chitin, which copolymerizes the building units of glucosamine and N-acetylglucosamine (GlcNAc). Thanks to the biologically fixed nitrogen in chitin's structure, marine biomass serves as a reservoir for not only renewable carbon but also nitrogen. Chitin and its monomers can be converted into many value-added chemicals, including N-containing furan derivatives, 5-hydroxymethylfurfural (HMF), protected amino acid, and acetic acid. Besides these chemicals, N-acetyl ethanolamine (NAEA) has gained an increased attention because ethanolamine (MEA, which can be produced by deacetylating NAEA) has numerous practical applications. For instance, ethanolamine is a feedstock in various industrial sectors (e.g., cosmetics and pharmaceuticals) and can be used for $CO_2$ capture. In this context, catalytic conversion of GlcNAc would be a key technology for producing sustainable NAEA from marine biomass. The production of NAEA from GlcNAc relied on a cascade reaction including retro-aldol condensation and subsequent hydrogenation of 2-acetamido-acetaldehyde intermediate (Scheme 1). However, achieving high NAEA yield is challenging because direct hydrogenation of GlcNAc is more favored than retro-aldol condensation in the presence of $H_2$, thus forming 2-acetamido-2-deoxysorbitol (r-GlcNAc) as the major product.

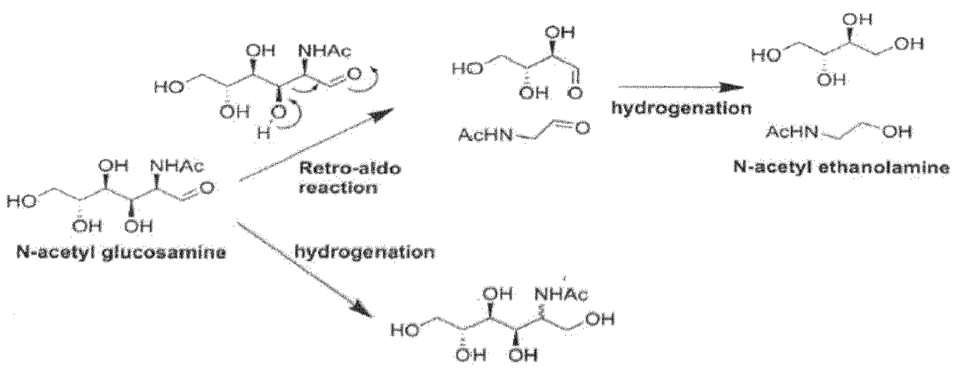


Scheme I- reaction pathway of N-acetyl ethanolamine

Bobbink et al. used NaOH and Ru/C to catalyze retro-aldol condensation and hydrogenation, respectively. An NMEA yield of 11% was obtained but uncleaved polyols were still the major products. Techikawara et. al. similarly used a metal-base (Ru/C-$NaHCO_3$) catalyst pair to facilitate the desirable cascade reaction. To further improve the NAEA yield, they lowered the $H_2$ pressure during the reaction to inhibit the rapid hydrogenation of GlcNAc and an increased yield of NAEA (15%) was obtained at 120°C. Both these prior arts relied on Ru-based catalyst and homogeneous base to produce NAEA from NAG. It would be challenging to produce NAEA using these processes in a large scale because of the use of costly Ru-based catalyst and the addition of homogeneous base catalyst, which requires further separation step for purifying products.

SUMMARY OF THE INVENTION

An objective of the present invention is to provide a method applicable to synthesize NAEA from GlcNAc without using homogeneous base as a co-catalyst.

In accordance with the foregoing and other objectives, one aspect of the disclosure provides a composite catalyst, including a basic LDHs (layered double hydroxides)-derived mixed oxide support and hydrogenation-active species on the basic LDHs-derived mixed oxide support. The composite catalyst can be prepared by calcination of LDHs

and then reduction under $H_2$ at a high temperature depending on the reducibility of the hydrogenation-active species, for example, at least about 700°C for Ni, and at least about 500°C for Cu. As the composite catalyst has both hydrogenation and base sites, retro-aldol condensation and subsequent hydrogenation of an intermediate produced from retro-aldol condensation can be catalyzed without necessitating the presence of a homogeneous base as a co-catalyst.

Another aspect of the disclosure provides a method for conversion of β-hydroxy carbonyl species, the method including: mixing β-hydroxy carbonyl species with the above-mentioned composite catalyst; and catalyzing retro-aldol condensation and then hydrogenation in hydrogen environment using the composite catalyst to complete conversion of the β-hydroxy carbonyl species. The β-hydroxy carbonyl species can be from biomass-derived feedstock, GlcNAc, and thus the disclosed method can be applied to produce an ethanolamine precursor (NAEA).

Yet another aspect of the disclosure provides a method for preparing an amino alcohol precursor, the method including: providing an amino saccharide; and mixing the amino saccharide with the above-mentioned composite catalyst for conversion of the amino saccharide into the amino alcohol precursor by retro-aldol condensation and then hydrogenation under catalysis of the composite catalyst. The step of providing the amino saccharide can include depolymerizing chitin biomass, and the weight ratio between the amino saccharide and the composite catalyst may be about 2 or less.

In the present invention, LDHs typically are doped $M^{3+}/N^{2+}$-LDHs, the $M^{3+}$ is a trivalent metal, the $N^{2+}$ is a bivalent metal, and the hydrogenation-active species are from doping elements of the doped $M^{3+}/N^{2+}$-LDHs. In one or more embodiments, the doped $M^{3+}/N^{2+}$-LDHs are Ni-doped hydrotalcite (HT) with the $M^{3+}$ and $N^{2+}$ being $Al^{3+}$ and $Mg^{2+}$, respectively, and the hydrogenation-active species are nickel nanoparticles. Accordingly, the earth-abundant composite catalyst (Ni, Al, and Mg) would be a highly favorable technology in circular economy. Herein, the total molar quantity of the doping elements and the $N^{2+}$ can be about 2 to about 4 times that of the $M^{3+}$. By calcination and then reduction, the Ni-doped HT can be converted into Ni/ hydrotalcite oxide (HTO), represented by $Ni/MgAlO_x$.

In the present invention, β-hydroxy carbonyl species may be one or more of β-hydroxyaldehyde and β-hydroxyketone, which can be from, for example, saccharide. The conversion of the β-hydroxy carbonyl species in the presence of the composite catalyst can be performed at a temperature in a range of about 60°C to about 120°C for about 1-4 hours. In one or more embodiments, the β-hydroxy carbonyl species includes a nitrogen-containing group at Cα position, and the conversion of the β-hydroxy carbonyl species allows for production of amino alcohol precursor. For example, the nitrogen-containing group may include *N*-acyl-substituted amino moiety, and the conversion can produce an alcohol compound containing an *N*-acyl-substituted amino moiety and having fewer carbon atoms than the β-hydroxy carbonyl species. The nitrogen atom of the nitrogen-containing group can be directly attached to the α-carbon of the β-hydroxy carbonyl species. In a particular example, the β-hydroxy carbonyl species is from an amino saccharide (such as GlcNAc), and the conversion involves first cleaving C2-C3 bond of GlcNAc by the basic support through retro-aldol condensation and then hydrogenating 2-acetamido-acetaldehyde intermediate by Ni nanoparticles, forming N-acetyl ethanolamine as the amino alcohol precursor (i.e. ethanolamine precursor). N-acetyl ethanolamine can be further deacetylated to form ethanolamine, which is an important bulk chemical that serves as an intermediate in various industrial sectors, including cosmetics, pharmaceuticals and $CO_2$ capture.

As used herein, the term "acyl" represents groups of 1, 2, 3, 4, 5, 6, 7, 8 or more carbon atoms of a straight, branched, cyclic configuration, saturated, unsaturated and aromatic, and combinations thereof, or hydrogen, attached to the parent molecular group through a carbonyl group. This group is exemplified by formyl (-C(O)H), acetyl (Ac or -C(O)Me), propionyl, isobutyryl, butanoyl, and the like.

As used herein, the term "high temperature" refers to temperatures greater than 300°C, and preferably greater than 400°C to 1000°C, such as 500°C-900°C, 600°C-900°C, 700°C-900°C, and 700°C-800°C.

As used herein, the term "about" is understood to account for minor increases and/or decreases beyond a recited value, which changes do not significantly impact the desired function of the parameter beyond the recited value(s). In some cases, "about" encompasses +/-10% of any recited value. As used herein, this term modifies any recited value, range of values, or endpoints of one or more ranges.

As used herein, the term "LDH (layered double hydroxide)" refers to a class of materials with positively charged layers and weakly bound charge-balancing anions located in the interlayer region and having the structural memory effect property which allows destroyed layered structures to be reconstructed into rehydrated LDHs under certain circumstance. At moderate calcination temperature, about 300-600 °C, the LDH can undergo thermal decomposition to metal oxide. The LDHs mentioned herein are not particularly limited, and may be any monometallic LDHs, multimetallic LDHs (e.g. binary, ternary, quaternary LDHs), metal-loaded LDHs or the like. The LDHs may be represented by the general formula: $M_x^{3+}N_{(1-x)}^{2+}\,(OH)_2A^{n-}yH_2O$, where $M^{3+}$ and $N^{2+}$ are trivalent and bivalent metal ions, respectively, and $A^{n-}$ is the interlayer ion of valence n. The x value represents the proportion of trivalent metal ion to the proportion of total amount metal ion and y denotes variable amounts of interlayer water. Common forms of LDH include $Mg^{2+}$ and $Al^{3+}$ as predominant metal species in a lattice of the LDH (i.e. $Al^{3+}/Mg^{2+}$-LDH, known as hydrotalcite) and $Mg^{2+}$ and $Fe^{3+}$ (i.e. $Fe^{3+}/Mg^{2+}$-LDH, known as pyroaurites). Further, additional metals other than the predominant metal species may be incorporated in the LDH to form metal-loaded $M^{3+}/N^{2+}$-LDH (such as Ni-loaded HT Cu-loaded HT, Ru-loaded HT and other metal-loaded HT).

As used herein, the term "saccharide" refers to sugars or sugar derivatives, polyhydroxylated aldehydes and ketones with an empirical formula that approximates $C_m(H_2O)_n$, i.e., wherein m and n are the same or about the same integer. The term is not intended to be limited to any saccharides and encompasses monosaccharides, disaccharides, oligosaccharides, polysaccharides and derivatives thereof (e.g. N-acetyl-glucosamine, glucosamine and any other amino saccharides).

As used herein, the term "or" is used to link alternatives provided within a list. The use of this term does not exclude the use of such alternatives together, such as in a combination; and the use of this term does not indicate or require that an alternative must be used alone. The use of this term can indicate that the alternative can be used alone or can be used together with any other alternative within the list.

As used herein, the phrase "one or more of A, B, and C" should be construed to mean a logical (A OR B OR C), using a non-exclusive logical OR, and should not be construed to mean "one or more of A, one or more of B, and one or more of C."

These and other features and advantages of the present invention will be further described and more readily apparent from the detailed description of the preferred embodiments which follows.

BRIEF DESCRIPTION OF THE DRAWINGS

FIG. 1 shows a schematic depiction of the conversion of GlcNAc into NAEA using a composite catalyst including a basic $MgAlO_x$ support and Ni nanoparticles.

FIG. 2 shows X-ray diffractogram of Ni/HTO-2Mg (catalyst A), Ni/HTO-3Mg (catalyst B) and Ni/HTO-4Mg (catalyst C).

FIG. 3 shows TEM image and particle size distribution of Ni/HTO-2Mg (a), Ni/HTO-3Mg (b) and Ni/HTO-4Mg (c).

FIG. 4 shows X-ray diffractograms of Ni/HT-2Mg, Ni/HTO-2Mg-600 (reduced at 600°C) and Ni/HTO-2Mg, in which the asterisk and cross signs denote the diffractions of metallic Ni and MgO, respectively.

FIG. 5 shows TPR profiles of NiO/HTO-nMg and NiO/MgO (a) and $CO_2$-TPD profiles of Ni/HTO-nMg and Ni/MgO (b).

FIG. 6 shows DRIFTS analysis of $CO_2$ chemisorbed on (a) Ni/HTO-2Mg (a) and Ni/MgO (b).

FIG. 7 shows catalytic results of GlcNAc conversion at 90 °C under 145 psi H2 for 1 h (a) and 4 h (b) using Ru/C-$NaHCO_3$ and bifunctional catalysts.

FIG. 8 shows $NH_3$-TPD results of Ni/HTO-nMg and Ni/MgO.

DETAILED DESCRIPTION OF THE PREFERRED EMBODIMENTS

Described herein is a bifunctional solid catalyst, which possesses both hydrogenation and base sites. Specifically, LDHs are used as a precursor to synthesize hydrogenation-active species (such as Ni catalysts) supported on basic LDHs-derived mixed oxides (such as magnesium-aluminum mixed oxide, $MgAlO_x$). The basic support enables base-catalyzed reactions to proceed on catalyst surface. This allows retro-aldol condensation prevails over direct hydrogenation at elevated temperature when surface basic sites outnumber hydrogenation sites. Accordingly, the LDHs-derived catalyst can be employed to produce NAEA from GlcNAc, as shown in FIG. 1. In addition to NAEA, other identified products, including ManNAc, r-GlcNA, r-ManNAc, and the diastereomers of 2-acetamido-2,3-dideoxyhexitol (expressed as deoxy-HexNAc), are formed during GlcNAc conversion through epimerization, direct hydrogentation, and dehydration, respectively (Scheme II).

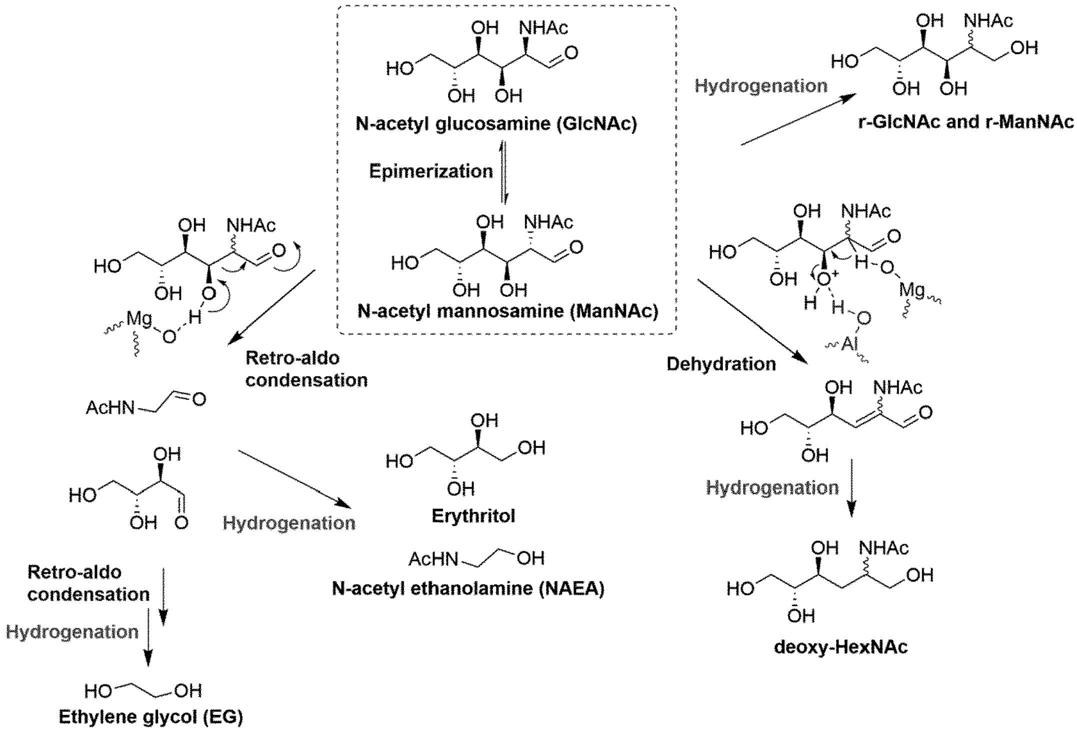


N-acetyl glucosamine (GlcNAc), N-acetyl mannosamine (ManNAc), r-GlcNAc and r-ManNAc, Erythritol, N-acetyl ethanolamine (NAEA), deoxy-HexNAc, Ethylene glycol (EG)

Scheme II- Proposed reaction network during GlcNAc conversion.

Experimental Details

*Chemicals and materials*

$Mg(NO_3)_2 \cdot 6H_2O$ (Alfa-Aesar, 98-102%), $Ni(NO_3)_2 \cdot 6H_2O$ (Alfa-Aesar, 98%) $Al(NO_3)_3 \cdot 9H_2O$ (Fisher Scientific, 98%), $Na_2CO_3$ (Sigma-Aldrich, 99.8%), $NaHCO_3$ (Sigma-Aldrich, ACS grade), NaOH (Shimakyu's Pure Chemicals, 99.4%), methanol (Macron Fine Chemicals, 99.8%), KBr (Sigma-Aldrich, IR grade) N-acetyl glucosamine (Sigma-Aldrich, 99%), N-Acetyl mannosamine (TCI chemical, 97%), Ethylene glycol (Vetec, 98%), meso-erythritol (TCI chemical, 99%), N-Acetyl ethanolamine (Combi-Blocks, 95%) and Ru/C catalyst (TCI chemical, wetted with 50% $H_2O$) were purchased and used without further purification. The water used in this study was deionized using a Labconco WaterPro Plus station. All gases were purchased from Air Product.

*Synthesis of LDHs-derived catalysts*

[Catalysts A-C]

The bifunctional composite catalyst containing Ni particles as hydrogenation-active species on a basic support was derived from LDHs synthesized using co-precipitation method. The LDHs were hydrotalcite materials whose Mg was partially substituted with Ni by adding nickel nitrate during co-precipitation. The molar ratio of Mg+Ni/Al was varied from 2 to 4 and a series of Ni-doped hydrotalcite-like precursors (Ni/HT-nMg, where n denotes Mg+Ni/Al, ranging from 2 to 4) whose general formula is $Mg_xNi_yAl_1CO_3(OH)_{16} \cdot 4H_2O$ (wherein x + y = n) was prepared by coprecipitation at controlled pH.

Typically, A mixture of metal nitrate solution was prepared by dissolving $Mg(NO_3)_2 \cdot 6H_2O$, $Ni(NO_3)_2 \cdot 6H_2O$ and $Al(NO_3)_3.9H_2O$ in 200 mL in a solvent pair containing 100 mL methanol and 100 mL water (MeOH-$H_2O$). The concentrations of $Mg^{2+}$, $Ni^{2+}$, and $Al^{3+}$ were 0.09 M, 0.01 M and 0.05 M, respectively. In parallel, an alkaline solution was prepared, which contains 0.2 M NaOH and 0.08 M $Na_2CO_3$, similarly in 200 mL MeOH-$H_2O$. Coprecipitation of Ni/HT-2Mg was proceeded by adding the metal nitrate solution into MeOH-$H_2O$ at a rate of 2 mL·min$^{-1}$ while regulating the pH value at 10 by adding the alkaline solution described above. Ni/HT-3Mg and Ni/HT-4Mg were prepared using the same procedure except the $Al^{3+}$ concentrations were varied (0.033 M for Ni/HT-3Mg and 0.025 M for Ni/HT-4Mg). After coprecipitation, the slurry was aged in a sealed polypropylene bottle at 65°C for 24 h in a conventional oven and the

resulting precipitate was collected by filtration. The crude Ni/HT-nMg was thoroughly washed with deionized water until the pH of the filtrate is below 8. The resulting paste was dried at 105°C for 15 h in a conventional oven. NiO/HTO-nMg was prepared by calcining the corresponding Ni/HT-nMg at 500°C for 12 h with a heating ramp of 2°C·min$^{-1}$ in a muffle furnace. Prior to activity measurement, NiO/HTO-nMg was reduced at 700°C for 5 h with a heating ramp of 2°C·min$^{-1}$ in a tubular furnace under a continuous flow of $H_2$. The activated catalysts, Ni/HTO-nMg, are denoted as catalyst A (Ni/HTO-2Mg), catalyst B (Ni/HTO-3Mg) and catalyst C (Ni/HTO-4Mg).

[Catalyst D]

The procedure as described earlier for catalyst A was repeated, except that $Ni(NO_3)_2{\cdot}6H_2O$ was replaced by $Cu(NO_3)_2{\cdot}6H_2O$. The reduction temperature was set to 500°C because of the better reducibility of Cu, which was confirmed by temperature programmed reduction analysis. The resulting catalyst is denoted as catalyst D.

*Synthesis of comparative catalysts*

[Catalyst E]

Ni/MgO was prepared using a modified method described above. In brief, a metal nitrate solution containing 0.015 M $Ni^{2+}$ and 0.2 M $Mg^{2+}$ was prepared by dissolving $Mg(NO_3)_2{\cdot}6H_2O$ and $Ni(NO_3)_2{\cdot}6H_2O$ in MeOH-$H_2O$. Coprecipitation was performed by adding this nitrate solution into MeOH/$H_2O$ at a rate of 2 mL· min$^{-1}$ while the pH value was controlled at 10 by using a 0.44 M NaOH in MeOH-$H_2O$. Then the aforementioned separation, washing and thermal treatment steps were repeated to obtain Ni/MgO, denoted as catalyst E.

[Catalyst F]

The procedure as described earlier for catalyst A was repeated without $Mg(NO_3)_2{\cdot}6H_2O$. The concentrations of $Ni^{2+}$ and $Al^{3+}$ were adjusted to 0.1 M and 0.033 M, respectively. The resulting catalyst is demoted as catalyst F.

[Catalyst G]

The 1.23 g $Ni(NO_3)_2{\cdot}6H_2O$ was dissolved in 5 mL deionized water. $WO_3$ was mixed with this solution and dried using a rotary evaporator (wet impregnation), Then the same dryig, calcination and reduction steps were repeated as catalyst A. The resulting catalyst is demoted as catalyst G.

*Catalyst characterization*

$N_2$ physisorption and $H_2$ chemisorption were conducted on a Micromeritics 3 Flex instrument. For $N_2$ physisorption, the samples were degassed and dried under reduced pressure at 150°C prior to measurement. The adsorption and desorption of $N_2$ were performed at -196°C cooled in liquid $N_2$. The specific surface area ($S_{BET}$) was determined using Brunauer-Emmett-Teller (BET) theory. For Hz chemisorption, the samples were first reduced at 400°C under $H_2$ for 1 h. Then the double isotherm method was performed, where the catalyst surface was first saturated by physisorbed and chemisorbed $H_2$ and then second adsorption was conducted after removing the physisorbed $H_2$ under reduced pressure. The amount of chemisorbed Hz was calculated by the subtraction method of two adsorption isotherms.

Temperature programmed reduction (TPR) as well as temperature programmed desorption of $CO_2$ and $NH_3$ ($CO_2$-TPD and $NH_3$-TPD) were conducted on a Micromeritics AutoChem II 2920. The samples were dried under He flow (30 mL·min$^{-1}$) at 500°C for 2 h prior these analyses. TPR was performed under the flow of 10% $H_2$ (20 mL·min$^{-1}$, balanced in Ar). The temperature was ramped (10°C· min$^{-1}$) from 50°C to 800°C while the consumption of $H_2$ was recorded by a thermal conductivity detector (TCD). For $CO_2$-TPD analysis, the sample was saturated with $CO_2$ by flowing 10% $CO_2$ (50 mL·min$^{-1}$, balanced in He) for 1 h at 40°C. The weakly adsorbed $CO_2$ was purged by switching $CO_2$ to He and then He was continuously flowed 1 h. Subsequently, the temperature was ramped (10°C·min$^{-1}$) from 40°C to 800°C while the desorption of $CO_2$ was recorded by a TCD. For $NH_3$-TPD analysis, the sample was saturated with $NH_3$ by flowing 10% $NH_3$ (30 mL ·min$^{-1}$, balanced in He) for 1 h at 35°C. The purging and desorption steps were the same as those of $CO_2$-TPD.

Powder X-ray diffraction (PXRD) was conducted on a Bruker D8 Advance X-ray diffractometer operated at 40 kV and 40 mA. Cu K$\alpha$ ($\lambda$ = 1.5406 Å) was used as a radiation source.

X-Ray Photoelectron Spectroscopy (XPS) measurements were carried out using a PHI Quanterall instrument with a scanning X-ray microprobe (Al anode). The beam size and power were 200 $\mu$m and 45 W, respectively. The pass energy was set to 55 eV to probe Mg 2p, Al 2p and Ni 2p with a step size of 0.1 eV

Nuclear Magnetic Resonance (NMR) was performed in a Bruker Avance 500MHz spectrometer with a TXI ($^1H/^{13}C/^{15}N$) 5mm CryoProbe with Z gradient.

The Ni particles of the catalysts were imaged using a high-resolution transmission electron microscopy (HR-TEM, JEM-2100F, JEOL, Japan) operated at 200 kV The specimen was prepared by dispersing the catalyst suspension (in ethanol) onto a carbon-lacey Cu grid (300 mesh) and dried at ambient temperature. Ni particle size was determined by sampling 50 Ni particles that have high contrast with $MgAlO_x$ support.

Diffuse reflectance Fourier transform infrared spectroscopy (DRIFTS) with $CO_2$ probe was measured on a Jasco FTIR 6700 instrument equipped with a Harrick Praying Mantis accessory. Sample was prepared by thoroughly mixing catalyst with KBr (catalyst/KBr = 1/60) using a mortar and a pestle. The physisorbed water was removed by heating the sample to 400°C under continuous $N_2$ flow (40 mL·min$^{-1}$) for 1 h. Sample was cooled down to 35°C and $CO_2$ was subsequently fed (40 mL· min$^{-1}$) into the chamber for 30 min. The weakly adsorbed $CO_2$ was purged by switching $CO_2$ to $N_2$ and then $N_2$ flow was maintained for 30 min. The DRIFTS spectra were recorded at 35 and 200 °C under a continuous flow of $N_2$.

*Conversion of amino saccharide*

The catalytic conversion of amino saccharide (GlcNAc) under reductive environment was performed in a Biotage Endeavor Pressure Reactor. Typically, 20 mg of catalyst was mixed with 4 mL GlcNAc(aq) (10 mg·mL$^{-1}$) in a glass tube. The solution was transferred into the pressure reactor. The headspace was purged and then the reactor vessel was pressurized with $H_2$ to 145 psi. The mixture was mechanically stirred with a turbine at 500 rpm and the reaction proceeded at a range between 60 °C and 90 °C. After the reaction was completed, the solid catalyst and soluble products were separated by centrifugation and filtration. The calculations of the measures for evaluating catalytic activity were shown below. ManNAc is used as an example to demonstrate the calculation of yield and selectivity. Other products were calculated in a similar way.

$$Conversion\ (X) = \frac{GlcNAc\ (t_0) - GlcNAc(t)}{GlcNAc\ (t_0)} \times 100\%$$

$$Yield_{ManNAc}\ (C\%) = \frac{C\ weight\ in\ ManNAc\ (t)}{C\ weight\ in\ GlcNAc\ (t_0)} \times 100\%$$

$$Selectivity_{ManNAc} = \frac{Yield_{ManNAc}}{Conversion} \times 100\%$$

Additionally, the conversion of GlcNAc into NAEA was also taken at 120°C to compare the yields of NAEA under Catalysts A-D and F-G.

*Product analysis and quantification*

The products were analyzed using a high-performance liquid chromatography (HPLC) equipped with a differential refractive index detector (RID). The filtered product mixture was analyzed using a Biorad Aminex HPX-87H column (300 mm × 7.8 mm, mobile phase: 0.01N $H_2SO_{4(aq)}$) with a flow rate of 0.6 mL min$^{-1}$ at 50°C. Because erythritol and GlcNAc are not resolved in HPX-87H column, GlcNAc in the product streams resulting from GlcNAc hydrogenation at 90°C using Ni/HTO-Mg and Ni/MgO (in which erythritol yield is above 5%) was quantified by a Shodex Ligand Exchange SP0810 Column (300 mm × 8.0 mm, mobile phase: $H_2O$) with a flow rate of 1 mL min$^{-1}$ at 70°C. GlcNAc, ManNAc, ethylene glycol, erythritol and NAEA were quantified using the peak areas generated by RID and calibration curves obtained from diluted analytical grade standards. For the quantification of erythritol, the unresolved peak was deconvoluted by substracting the theoretical peak area of GlcNAc from the area of the overlapped peak. The theoretical peak area of GlcNAc was estimated by using its quantity (through analysis using Shodex SP0810 Column) and the calibration curve for HPX-87H column. The analytical grade standards of r-ManNAc, r-GlcNAc and HexNAc were not commercially available, so their calibration curves were produced using self-prepared standards and the preparation methods were described below.

*Purification of rManNAc, r-GlcNAc and deoxy-HexNAc for HPLC calibration*

The r-ManNAc and r-GlcNAc standards were prepared by hydrogenating GlcNAc and ManNAc, respectively using Ru/C at 90 °C for 1 h under 145 psi $H_2$. The mass ratio of catalyst and reactant was maintained at 1/2. Because Ru/C hydrogenates GlcNAc and ManNAc with 100% selectivity, the standards of r-ManNAc and r-GlcNAc can be obtained through lyophilizing the filtered product solution without further purification.

For deoxy-HexNAc, GlcNAc hydrogenation was performed at 90 °C for 4 h under 145 psi $H_2$ using Ni/HTO-2Mg (mass of catalyst/mass of GlcNAc = 1/2). The fully reacted solution was lyophilized, resulting in a viscous liquid mixture. The mixture was redissolved in methanol and purified using silica gel column chromatography with methanol and chloroform (methanol/chloroform = 1/2). The separated products were tracked through thin-layer chromatography (TLC) with pre-coated glass plates of silica gel 60 F254 (0.25 mm, E. Merck). The spots were visualized by dipping the TLC plate into Hanessian's Stain (cerium ammonium molybdate) and subsequently heating it on a hot plate. The purified product was dried under reduced pressure at room temperature and its major components were identified as the diastereomeric isomers of 2-Acetamido-2,3-dideoxyhexitols (deoxy-HexNAc), whose structures were confirmed using $^{13}C$ and $^{1}H$ NMR spectroscopy as well as electrospray ionization mass spectrometry (ESI-MS). The second most abundant component is erythritol, which was identified through HPLC analysis by means of its known retention time. The rest of minor species only account for approximately 5% of the total RID peak area in the chromatograph. Accordingly, the actual weight of the self-prepared deoxy-HexNAc standard was determined by subtracting the erythritol weight (estimated using the peak area in the resulting chromatogram of the diluted standard) from the pre-measured weight and then multiply the resulting value by 0.95. A series of diluted deoxy-HexNAc standards was prepared and analyzed for obtaining the calibration curve of deoxy-HexNAc.

Result and discussion

*Catalyst characterization*

The calcined Ni/HT-nMg (NiO/HTO-nMg) were thermally reduced with continuous flow of $H_2$, resulting in Ni/HTO-nMg, which comprises Ni particles supported on $MgAlO_x$ (HTO). The appearance of X-ray diffraction at 44 degree indicates the formation of Ni nanoparticles after these thermal treatments (FIG. 2). TEM analyses showed that the Ni particle size of Ni/HTO-nMg ranges from 4 to 5 nm (FIG. 3; Ni/HTO-2Mg : 4.3 $\pm$ 1.1 nm, Ni/HTO-3Mg : 4.6 $\pm$ 1.1 nm, Ni/HTO-4Mg : 4.2 $\pm$ 0.9 nm). XRD analyses also suggested that thermal treatments transformed LDH structure into segregated phases containing Ni particles and $MgAlO_x$ (FIG. 4, uppermost and lowermost lines). Temperature-programmed reduction (TPR) profiles indicated that the onset of the reduction of NiO/HTO-nMg is around 700 °C (FIG. 5a). In comparison, the reduction of NiO/MgO (prepared similarly using co-precipitation method followed by calcination) began at 500 °C. Agreeing with previous reports, $Ni^{2+}$ was likely incorporated into LDH's crystal structure, thus lowering its reducibility. Indeed, when NiO/HTO-2Mg was reduced at 600 °C, the diffraction peak of Ni was absent (FIG. 4, middle line) and the resulting catalyst showed a poor activity. Therefore, all the catalysts used in this study were reduced at 700 °C for 5 h prior to activity assessments.

The surface basicity of Ni/HTO-nMg and Ni/MgO were analyzed and quantified by temperature-programmed desorption using $CO_2$ as a probe molecule ($CO_2$-TPD). All catalysts show a broad desorption band at the temperature range of 100 to 300 °C (FIG. 5b), which can be attributed to medium basic sites. For Ni/HTO-nMg, an additional desorption band can be observed at above 600 °C, suggesting the existence of stronger basic sites. The absence of stronger basic site on Ni/MgO implied that the basicity of some Mg species might be enhanced when Mg coordinates with Al. To confirm that $CO_2$ chemisorb on basic sites, *in-situ* diffuse reflectance infrared Fourier transform spectroscopy (DRIFTS) was conducted (FIG. 6). After $CO_2$ adsorption, monodentate $CO_3$* and $HCO_3$* were found on both Ni/HTO-2Mg and Ni/MgO. It was reported that $CO_3$* resulted from $CO_2$ interacting with medium and strong basic sites (e.g., Mg-O moiety). In comparison, $HCO_3$* formed on weaker basic sites (e.g., Mg-OH moiety) and it desorbed at 200 °C. These characterization results substantiate the surface bifunctionality of Ni/HTO-nMg and Ni/MgO whose hydrogenation and basic sites might enable the cascade reaction of GlcNAc.

The characterization results of Ni-based catalysts were summarized in below Table 1. The analyses of ICP-Mass, $N_2$ physisorption, XPS, static $H_2$ chemisorption, $CO_2$-TPD and $NH_3$-TPD showed Ni loading of about 6-10%, $S_{BET}$ of about 200-250 $m^2$/g, surface Mg/Al ratio of about 1-3, about 50-75 $\mu$mol/g, about 700-850 $\mu$mol/g and about 100-250 $\mu$mol/g, respectively.

[Table 1]

| Catalyst | Loading (Ni-%) | $S_{BET}$ ($m^2$/g) | Mg/Al ratio | Chemisorbed $H_2$ (μmol/g) | $CO_2$-TPD (μmol/g) | $NH_3$-TPD (μmol/g) |
|---|---|---|---|---|---|---|
| A | 6.9 | 216.6 | 1.7 | 52 | 731 | 223 |
| B | 7.3 | 237.7 | 2.5 | 57 | 728 | 195 |
| C | 9.3 | 241.9 | 2.9 | 73 | 801 | 156 |
| E | 19.6 | 128.1 | N.A. | 74 | 402 | 40 |

*Conversion of amino saccharide*

Amino saccharide (GlcNAc) is converted at elevated temperatures similarly in $H_2$ environment using Ni/HTO-nMg, Ni/MgO, and Ru/C-$NaHCO_3$ to study the effects of surface bifunctionality on retro-aldol condensation and the resulting formation of ethanolamine precursor (NAEA). The formation of NAEA was confirmed by analyzing the separated fraction using a nuclear magnetic resonance spectroscopy and mass spectrometry. The $^1$H NMR (400MHz, $D_2O$) showed the representative chemical shifts of NAEA (1.8 (s, 3H), 3.2 (t, 2H), 3.52 (t, 2H), 8.0 (s, H)), and $[M+H]^+$=104 was detected using mass spectrometry with eleetrospray ionization.

It was found that Ni/HTO-nMg can achieve fairly high NAEA yield at 90 °C, which is lower than most of the reported conditions. At 90 °C under 145 psi $H_2$, Ru/C-$NaHCO_3$ exhibited the highest selectivity towards r-GlcNAc (48.9%, FIG. 7a) among all tested catalysts, which can be partially attributed to the stronger hydrogenation strength of Ru and its smaller particle size ($d_{Ru}$ = 1.8 ± 0.4 nm). Additionally, the weaker basicity of $NaHCO_3$ leads to slower retro-aldol condensation, thus lowering the selectivity towards NAEA (7.8%). The other identified products are ManNAc, r-ManNAc, and the diastereomers of 2-acetamido-2,3-dideoxyhexitol (expressed as deoxy-HexNAc).

Stark contrast can be seen when comparing the selectivities between Ru/C-$NaHCO_3$ and Ni/HTO-nMg (FIG. 7a). Ni/HTO-nMg produced little r-GlcNAc and r-ManNAc (selectivities towards both products were below 5%), indicating hydrogenation of GlcNAc and ManNAc became side reaction when using Ni/HTO-nMg. The major components produced by Ni/HTO-nMg were deoxy-HexNAc and NAEA. This result substantiated that retro-aldol condensation as well as dehydration prevail over direct hydrogenation of GlcNAc and ManNAc over Ni/HTO-nMg. The drastic change of reaction pathway can be attributed to basic sites on HTO, which promotes both retro-aldol condensation and dehydration. Since Ni/MgO also has a basic MgO support, its selectivities towards deoxy-HexNAc (10.4%) and NAEA (15.4%) are higher than those of Ru/C-$NaHCO_3$, but intriguingly, lower than those of Ni/HTO-nMg. This phenomenon is ascribed to the distinct surface characteristics of Ni/MgO and Ni/HTO-nMg. It can be found that the number of surface basic sites of Ni/MgO is significantly smaller than those of Ni/HTO-nMg, which supposedly results from its lower surface area ($CO_2$-TPD and $S_{BET}$, Table 1). Moreover, Ni/MgO lacks strong basic sites according to $NH_3$-TPD analysis (FIG. 5b). With fewer and weaker basic sites on Ni/MgO, retro-aldol condensation and dehydration compete with hydrogenation to a less extent, thus leading to higher selectivities towards r-GlcNAc (17.0%) and r-ManNAc (12.7%). These results conclude that both retro-aldol condensation and dehydration are surface-catalyzed and underscored the importance of basic support that could be adapted for controlling the reaction pathway during GlcNAc conversion.

To maximize the NAEA yield, the reaction was prolonged to 4 h using Ni/HTO-nMg and Ni/MgO to reach nearly full conversion (conversion > 95%). Ni/HTO-nMg produced NAEA with yields higher than 19% (FIG. 7b). Particularly, Ni/HTO-4Mg resulted in an NAEA yield of 21%, which is, to the best of our knowledge, the highest NAEA yield obtained at the lowest temperature (90°C) to date. In comparison, Ni/MgO resulted in a slightly lower NAEA yield (17%) because of more prevalent hydrogenation of GlcNAc and ManNAc. To summarize the reaction pathway, GlcNAc undergoes four parallel reactions at 90°C, including epimerization, direct hydrogenation, dehydration and retro-aldol condensation. The selectivities towards final products were governed by how the later three reactions compete with each other. Direct hydrogenation of GlcNAc is the main route when Ru/C-$NaHCO_3$ is used, leading to the highest selectivity towards r-GlcNAc. In the presence of solid base (MgO and HTO), both dehydration and retro-aldol condensation are facilitated, thus increasing the selectivities towards NAEA and deoxy-HexNAc whereas hydrogenation of GlcNAc and ManNAc is suppressed.

Additionally, formation rates of deoxy-HexNAc and NAEA during GlcNAc conversion were measured at 60°C and listed in Table 2.

[Table 2]

| Catalysts | deoxy-HexNAc (umol/h) | NAEA (umol/h) |
|---|---|---|
| A | 4.4 ± 0.2 | 3.9 ± 0.4 |
| B | 5.2 ± 0.4 | 5.8 ± 0.4 |

(continued)

| Catalysts | deoxy-HexNAc (umol/h) | NAEA (umol/h) |
|---|---|---|
| C | 4.1 ± 0.4 | 5.0 ± 0.8 |
| E | 2.3 ± 0.4 | 2.7 ± 0.4 |

It can be unexpectedly found that the formation of deoxy-HexNAc on Ni/HTO-4Mg is the slower (4.1 umol/h) than on Ni/HTO-2Mg (4.4 umol/h). Ni/HTO-4Mg also resulted in a lower selectivity towards deoxy-HexNAc at 90°C (FIG. 7a). These results indicate that dehydration is catalyzed by other active species beyond base, considering Ni/HTO-4Mg has the highest number of basic site (Table 1). Surface acid is then tentatively proposed as the additional active site for dehydrating HexNAc because dehydration is typically initiated by protonating hydroxyl group. Accordingly, $NH_3$-TPD was performed to quantify the acid sites on Ni/HTO-nMg (FIG. 8) and their quantities were found to be inversely correlated with the Mg/Al ratio (Table 1). The increased number of acid site likely results from more exposed Al-OH on the surface when Mg/Al ratio decreases.

It can be concluded that the key to selectively producing NAEA from GlcNAc is promoting retro-aldol condensation while suppressing direct hydrogenation and dehydration. Retro-aldol condensation is spontaneously favored at elevated temperatures provided that the catalyst surface is enriched with basic sites. However, higher temperatures also facilitate dehydration, which competes with retro-aldol condensation and limits the yield of NAEA. Dehydration can be inhibited through reducing the surface acidity by increasing the Mg/Al ratio. With less acid sites, the concerted synergy of hydrogenation and basic sites of Ni/HTO-4Mg led to the highest NAEA yield in this study.

Moreover, the yields of NAEA at 120°C using catalysts A-D and F-G were listed in Table 3.

[Table 3]

| Catalysts | $Yield_{NAEA}$ at 120°C (% of C yield) |
|---|---|
| A | 17.1 |
| B | 21.5 |
| C | 8.6 |
| D | 7.0 |
| F | 1.3 |
| G | 0 |

Conclusion

Ni-doped hydrotalcite-like material can be used as a precursor to synthesize a series of bifunctional Ni/HTO-nMg catalysts, comprising a basic MgAlOx support and Ni nanoparticles. At elevated temperatures, the abundant basic sites on HTO allow retro-aldol condensation to outpace direct hydrogenation of GlcNAc, thus achieving an exceptional selectivity towards NAEA. In comparison, Ni/MgO is more selective towards r-GlcNAc because it has fewer surface basic sites. Additionally, dehydration of GlcNAc is identified as another competing reaction, which forms deoxy-HexNAc. The selectivity towards deoxy-HexNAc is inversely correlated with the Mg/Al ratio because dehydration is promoted by not only basic but also acid sites. Therefore, Ni/HTO-4Mg outperforms all tested catalysts, resulting in the highest NAEA yield of 21% at 90°C.

The above examples are intended for illustrating the embodiments of the subject invention and the technical features thereof, but not for restricting the scope of protection of the subject invention. Many other possible modifications and variations can be made without departing from the scope of the invention as hereinafter claimed. The scope of the subject invention is based on the claims as appended.

## Claims

**1.** A method for conversion of β-hydroxy carbonyl species, the method comprising:

mixing β-hydroxy carbonyl species with a composite catalyst derived from layered double hydroxides (LDHs), wherein the composite catalyst includes a basic LDHs-derived mixed oxide support and hydrogenation-active species on the basic LDHs-derived mixed oxide support; and

catalyzing retro-aldol condensation and then hydrogenation in hydrogen environment using the composite catalyst to complete conversion of the β-hydroxy carbonyl species.

2. The method of the previous claim, wherein the β-hydroxy carbonyl species includes a nitrogen-containing group or wherein the β-hydroxy carbonyl species includes a nitrogen-containing group at Cα position, and wherein preferably the nitrogen-containing group includes an *N*-acyl-substituted amino moiety.

3. The method of anyone of the previous claims, wherein the LDHs are doped $M^{3+}/N^{2+}$-LDHs, the $M^{3+}$ is a trivalent metal, and the $N^{2+}$ is a bivalent metal, and wherein preferably the hydrogenation-active species are from doping elements of the doped $M^{3+}/N^{2+}$-LDHs, and the total molar quantity of the doping elements and the $N^{2+}$ is 2 to 4 times that of the $M^{3+}$.

4. The method of the previous claim, wherein the $M^{3+}$ is $Al^{3+}$, and the $N^{2+}$ is $Mg^{2+}$.

5. The method of anyone of the previous claims, wherein the hydrogenation-active species are nickel nanoparticles, and/or wherein the conversion of the β-hydroxy carbonyl species produces an alcohol compound containing an *N*-acyl-substituted amino moiety and having fewer carbon atoms than the β-hydroxy carbonyl species.

6. The method of anyone of the previous claims, wherein the β-hydroxy carbonyl species is from a saccharide, and wherein preferably the saccharide is monosaccharide, and/or wherein the conversion of the β-hydroxy carbonyl species proceeds at a temperature in a range of 60°C to 120°C.

7. A method for preparing an amino alcohol precursor, comprising:

   providing an amino saccharide; and
   mixing the amino saccharide with a composite catalyst derived from layered double hydroxides (LDHs) for conversion of the amino saccharide into the amino alcohol precursor by retro-aldol condensation and then hydrogenation under catalysis of the composite catalyst, wherein the composite catalyst includes a basic LDHs-derived mixed oxide support and hydrogenation-active species on the basic LDHs-derived mixed oxide support.

8. The method of the previous claim, wherein the amino saccharide includes an *N*-acyl-substituted amino moiety.

9. The method of any one of the two previous claims, wherein the LDHs are doped $M^{3+}/N^{2+}$-LDHs, the $M^{3+}$ is a trivalent metal, and the $N^{2+}$ is a bivalent metal, and wherein preferably the hydrogenation-active species are from doping elements of the doped $M^{3+}/N^{2+}$-LDHs, and the total molar quantity of the doping elements and the $N^{2+}$ is 2 to 4 times that of the $M^{3+}$.

10. The method of the previous claim, wherein the $M^{3+}$ is $Al^{3+}$, and the $N^{2+}$ is $Mg^{2+}$.

11. The method of anyone of the previous four claims, wherein the hydrogenation-active species are nickel nanoparticles, and/or wherein amino alcohol precursor is an alcohol compound containing an *N*-acyl-substituted amino moiety and having fewer carbon atoms than the amino saccharide, and/or wherein the amino saccharide is N-acetyl glucosamine.

12. The method of anyone of the previous five claims, wherein the conversion of the amino saccharide into the amino alcohol precursor proceeds in hydrogen environment at a temperature in a range of 60°C to 120°C, and/or wherein a weight ratio between the amino saccharide and the composite catalyst is 2 or less.

13. The method of anyone of the previous six claims, wherein the amino saccharide is N-acetyl glucosamine, and the amino alcohol precursor is N-acetyl ethanolamine, and/or wherein the step of providing the amino saccharide includes depolymerizing chitin biomass.

14. A composite catalyst derived from layered double hydroxides (LDHs), comprising:

   a basic LDHs-derived mixed oxide support, wherein the LDHs are doped $M^{3+}/N^{2+}$-LDHs, the $M^{3+}$ is a trivalent metal, and the $N^{2+}$ is a bivalent metal; and
   hydrogenation-active species on the basic LDHs-derived mixed oxide support, wherein the hydrogenation-active species are from doping elements of the doped $M^{3+}/N^{2+}$-LDHs, and the total molar quantity of the doping elements and the $N^{2+}$ is 2 to 4 times that of the $M^{3+}$.

**15.** The composite catalyst of the previous claim, wherein the $M^{3+}$ is $Al^{3+}$, and the $N^{2+}$ is $Mg^{2+}$ and/or wherein the hydrogenation-active species are nickel nanoparticles.

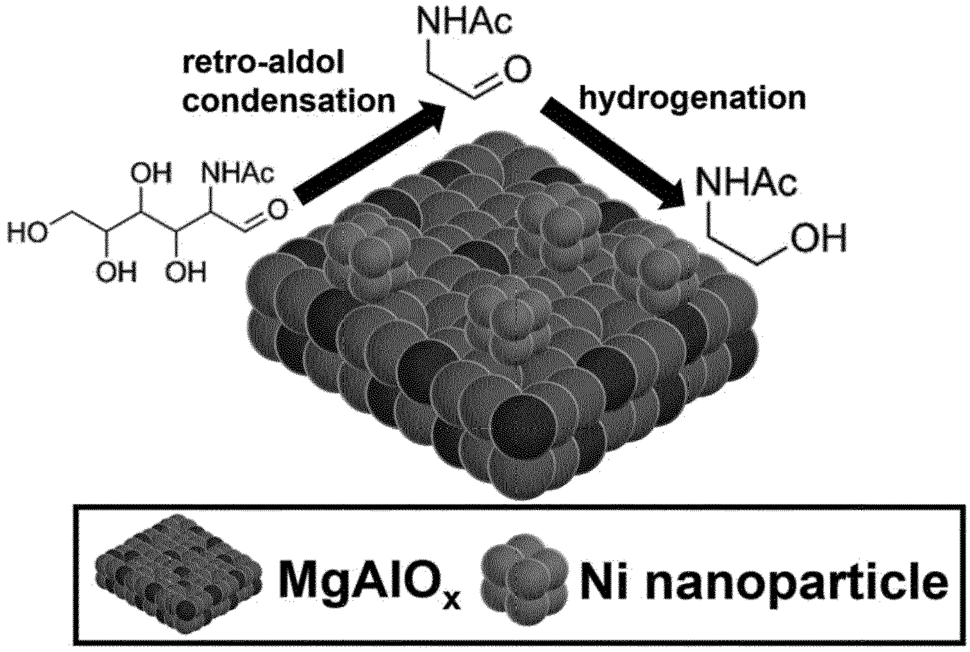
retro-aldol condensation
hydrogenation
MgAlO$_x$
Ni nanoparticle

FIG. 1

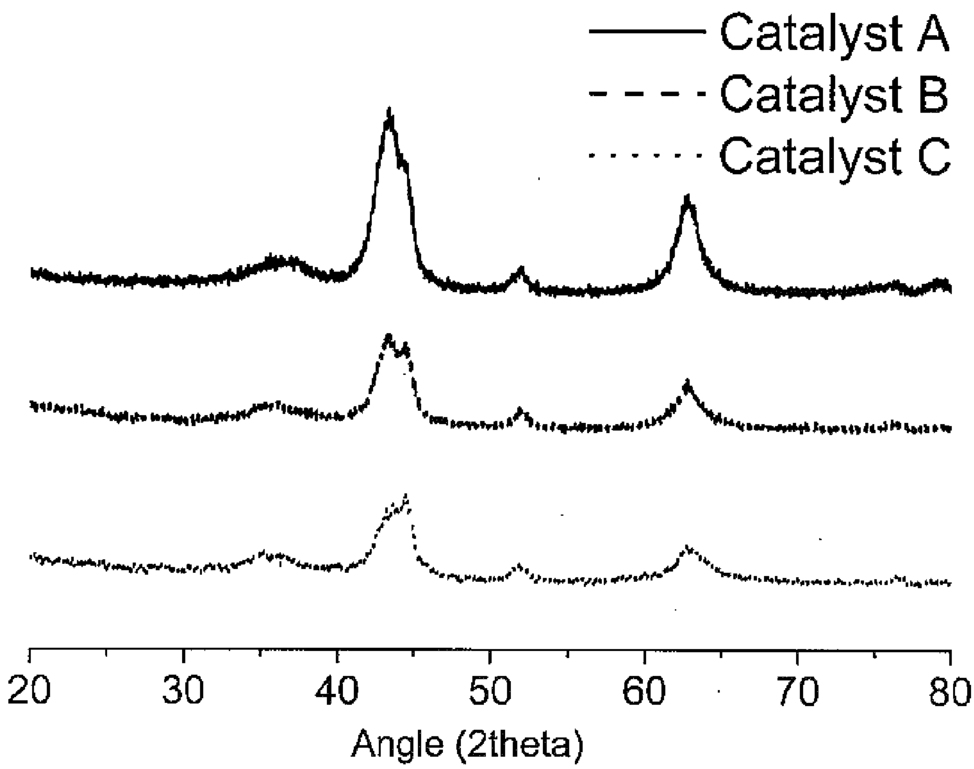
Catalyst A
Catalyst B
Catalyst C
20
30
40
50
60
70
80
Angle (2theta)

FIG. 2

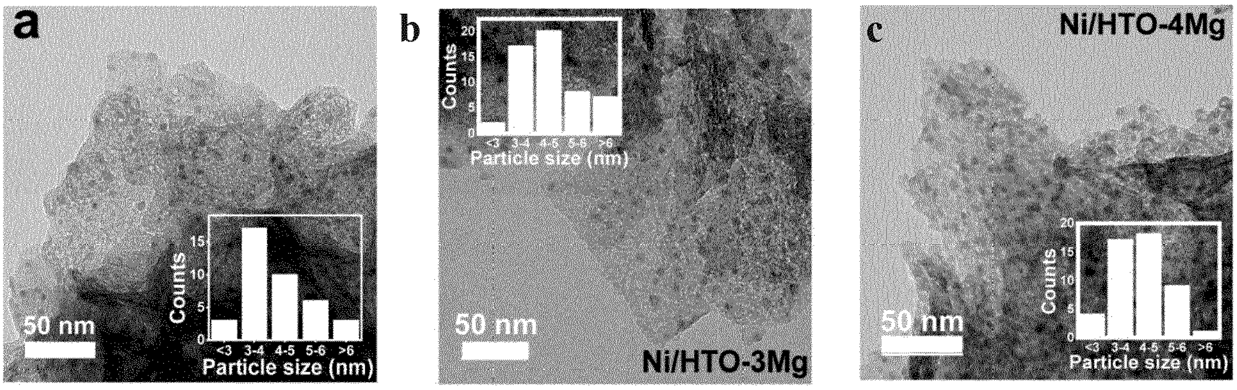


FIG. 3

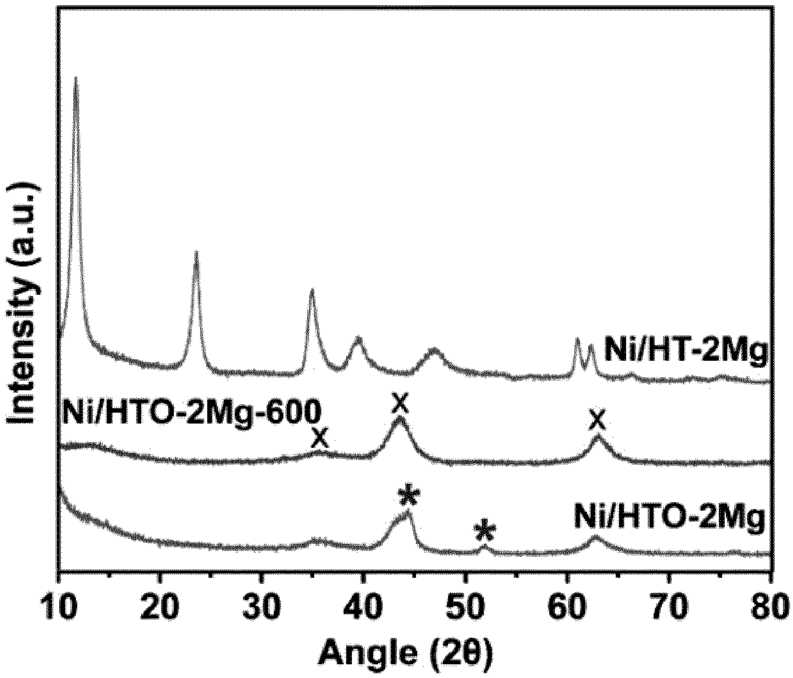
Intensity (a.u.)
Ni/HT-2Mg
Ni/HTO-2Mg-600
Ni/HTO-2Mg
10 20 30 40 50 60 70 80
Angle (2θ)

FIG. 4

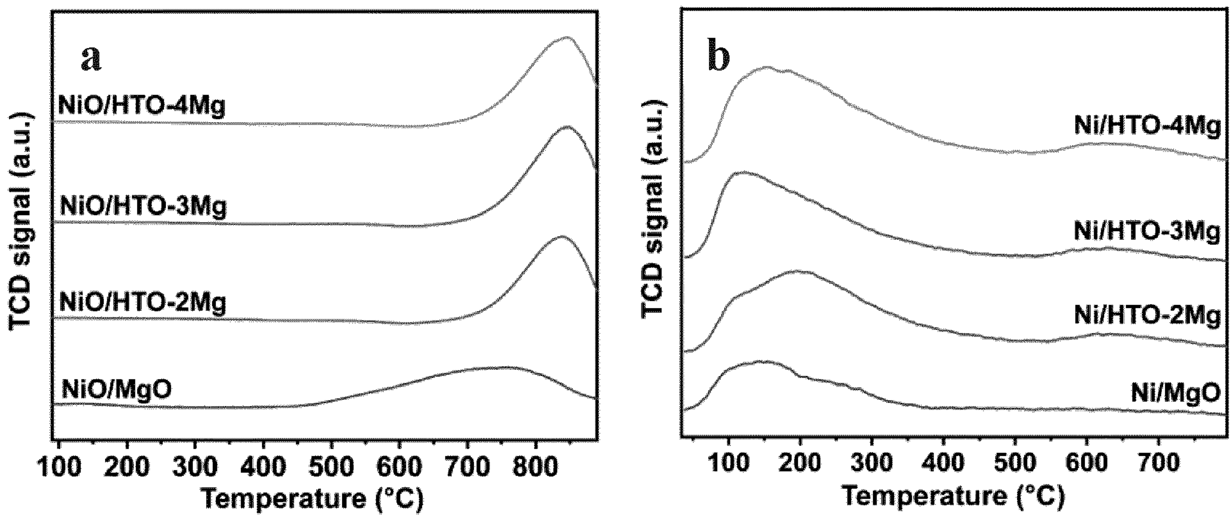
a
NiO/HTO-4Mg
NiO/HTO-3Mg
NiO/HTO-2Mg
NiO/MgO
TCD signal (a.u.)
100 200 300 400 500 600 700 800
Temperature (°C)
b
Ni/HTO-4Mg
Ni/HTO-3Mg
Ni/HTO-2Mg
Ni/MgO
TCD signal (a.u.)
100 200 300 400 500 600 700
Temperature (°C)

FIG. 5

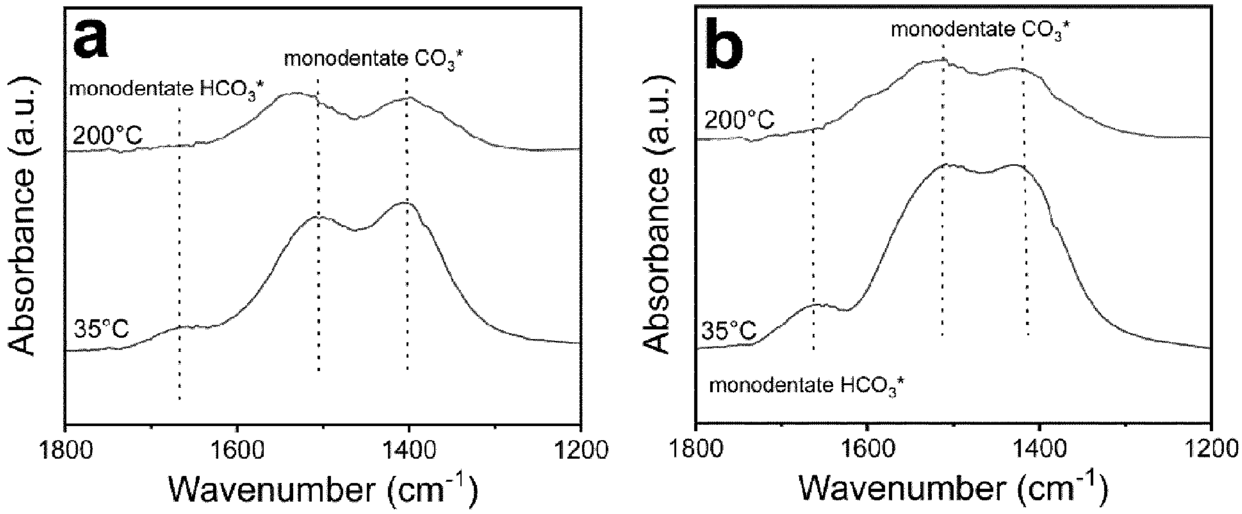
a
monodentate HCO$_3$*
monodentate CO$_3$*
200°C
35°C
Absorbance (a.u.)
1800
1600
1400
1200
Wavenumber (cm$^{-1}$)
b
monodentate CO$_3$*
200°C
35°C
monodentate HCO$_3$*
Absorbance (a.u.)
1800
1600
1400
1200
Wavenumber (cm$^{-1}$)

FIG. 6

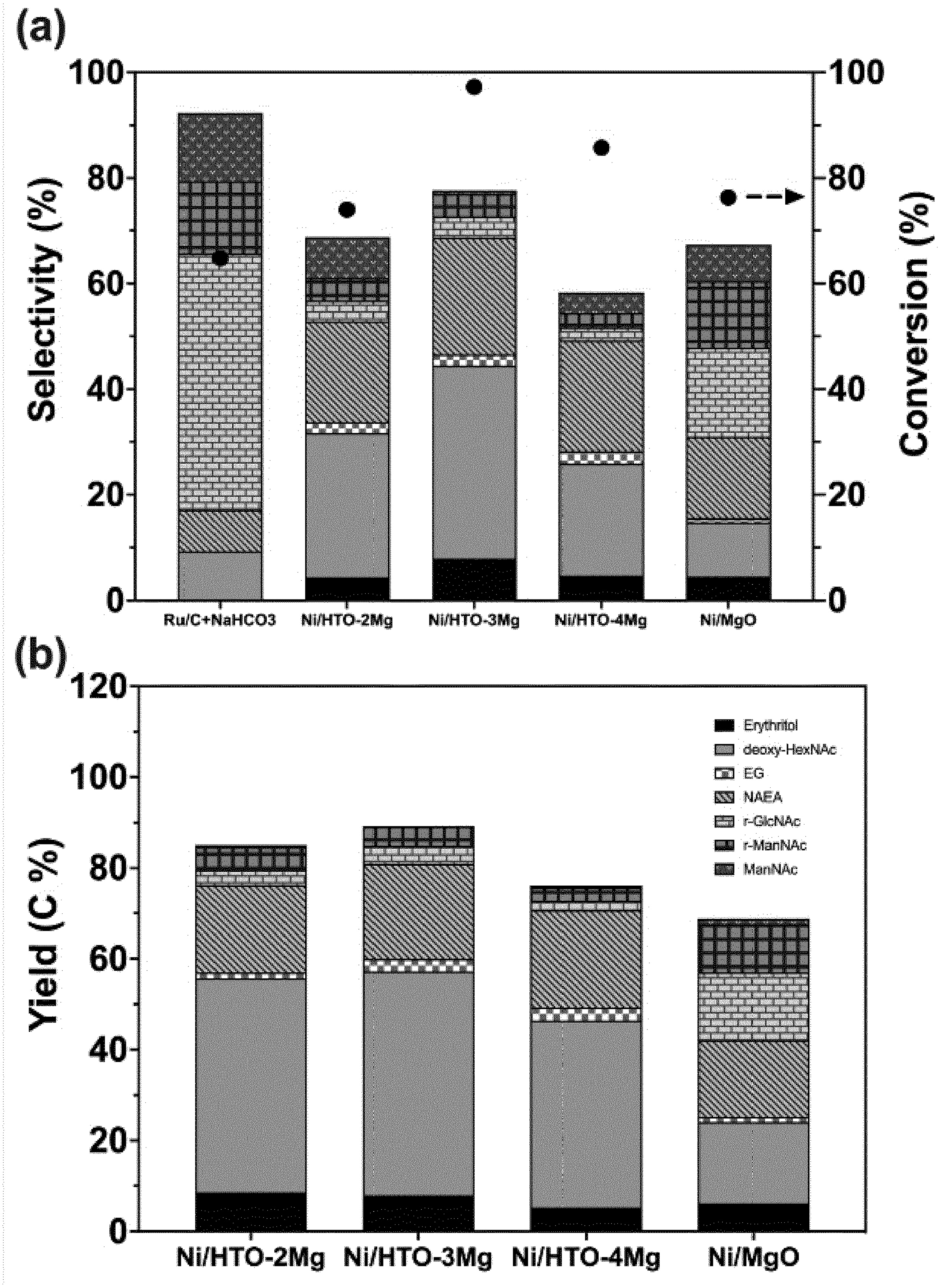
(a)
Selectivity (%)
Conversion (%)
100
80
60
40
20
0
Ru/C+NaHCO3
Ni/HTO-2Mg
Ni/HTO-3Mg
Ni/HTO-4Mg
Ni/MgO
(b)
Yield (C %)
120
100
80
60
40
20
0
Erythritol
deoxy-HexNAc
EG
NAEA
r-GlcNAc
r-ManNAc
ManNAc
Ni/HTO-2Mg
Ni/HTO-3Mg
Ni/HTO-4Mg
Ni/MgO

FIG. 7

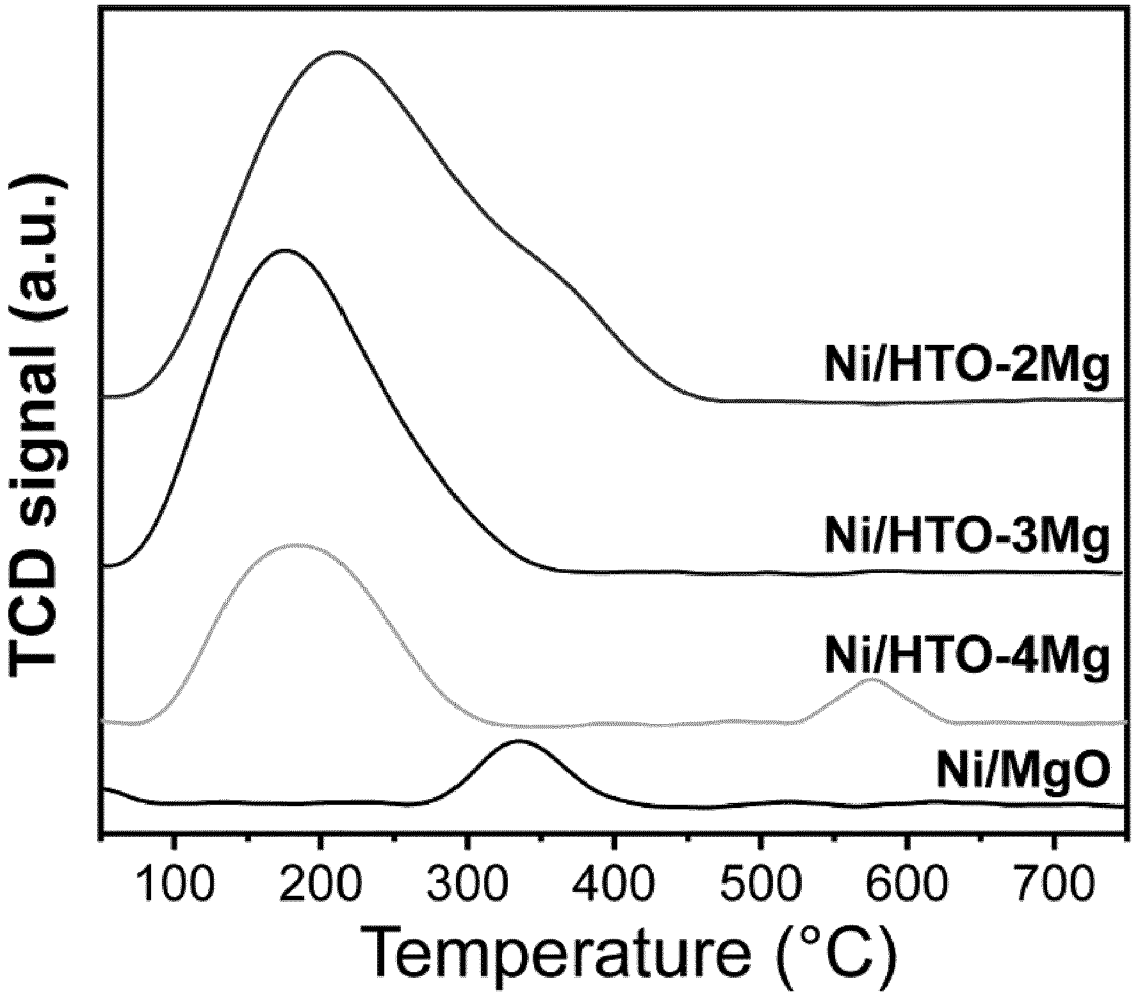
TCD signal (a.u.)
Ni/HTO-2Mg
Ni/HTO-3Mg
Ni/HTO-4Mg
Ni/MgO
100
200
300
400
500
600
700
Temperature (°C)

FIG. 8

Europäisches Patentamt
European Patent Office
Office européen des brevets

# EUROPEAN SEARCH REPORT

Application Number
EP 24 15 4926

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MA PENGFEI ET AL: "Insight into the function of base-promoted Cu-containing catalysts for highly efficient hydrogenolysis of cellulose into polyols", JOURNAL OF ENERGY CHEMISTRY, ELSEVIER, AMSTERDAM, NL, vol. 25, no. 5, 27 July 2016 (2016-07-27), pages 782-792, XP029754994, ISSN: 2095-4956, DOI: 10.1016/J.JECHEM.2016.07.001 | 1,3-6, 14,15 | INV. B01J23/755 B01J35/73 B01J35/45 |
| Y | * page 783, right-hand column, paragraph 2.1; table 2 * * Scheme 1; page 784, left-hand column, paragraph 2.3 - column 1, paragraph 1; figure 8 * ----- | 2,7-13 | |
| X | LI XUEMEI ET AL: "Efficient Synthesis of 5-Amino-1-pentanol from Biomass-Derived Dihydropyran over Hydrotalcite-Based Ni-Mg3AlOx Catalysts", ACS SUSTAINABLE CHEMISTRY & ENGINEERING, vol. 8, no. 16, 3 April 2020 (2020-04-03), pages 6352-6362, XP093175907, US ISSN: 2168-0485, DOI: 10.1021/acssuschemeng.0c00394 * abstract; page 6352 * * page 6353, right-hand column, paragraph 3; table 1 * * page 6354, right-hand column, paragraph 1 * * Scheme 1; page 784, left-hand column, paragraph 2.3 - right-hand column, paragraph 1 * -/-- | 14,15 | TECHNICAL FIELDS SEARCHED (IPC): B01J C07C |

The present search report has been drawn up for all claims

1

| Place of search | Date of completion of the search | Examiner |
|---|---|---|
| Munich | 18 June 2024 | Beckmann, Oliver |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 24 15 4926

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | -& Li Xuemei ET AL: "Supporting Information - Efficient synthesis of 5-amino-1-pentanol from biomass-derived dihydropyran over hydrotalcite-based Ni-Mg3AlOx catalysts", ACS Sustainable Chemistry & Engineering, 3 April 2020 (2020-04-03), pages S1-S11, XP093175908, Retrieved from the Internet: URL:https://doi.org/10.1021/acssuschemeng.0c00394 * page S2, paragraph 2 * ----- | 14,15 | |
| X | CUELLO-PENALOZA PAOLO A ET AL: "Ethanol to distillate-range molecules using Cu/MgxAlOy catalysts with low Cu loadings", APPLIED CATALYSIS B. ENVIRONMENTAL, ELSEVIER, AMSTERDAM, NL, vol. 304, 3 December 2021 (2021-12-03), XP086918810, ISSN: 0926-3373, DOI: 10.1016/J.APCATB.2021.120984 [retrieved on 2021-12-03] * page 2, right-hand column, paragraph 2 * * page 2, column 2, paragraph 2.1 * * page 4; table 1 * ----- | 14,15 | TECHNICAL FIELDS SEARCHED (IPC) |

-/--

1

The present search report has been drawn up for all claims

| Place of search | Date of completion of the search | Examiner |
|---|---|---|
| Munich | 18 June 2024 | Beckmann, Oliver |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 24 15 4926

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TECHIKAWARA KOTA ET AL: "Conversion of N-Acetylglucosamine to Protected Amino Acid over Ru/C Catalyst", ACS SUSTAINABLE CHEMISTRY & ENGINEERING, vol. 6, no. 9, 9 August 2018 (2018-08-09), pages 12411-12418, XP093175878, US ISSN: 2168-0485, DOI: 10.1021/acssuschemeng.8b02951 * page 12412, left-hand column, paragraph 2; figures 2,3 * * page 12412, left-hand column, paragraph 3 - right-hand column, paragraph 1 * ----- | 2,7-13 | |

TECHNICAL FIELDS SEARCHED (IPC)

The present search report has been drawn up for all claims

1

| Place of search | Date of completion of the search | Examiner |
|---|---|---|
| Munich | 18 June 2024 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3